# EUROPEAN PATENT APPLICATION

(11) **EP 2 283 865 A1**
(43) Date of publication of application: **16.02.2011**
(21) Application number: 10184108.8
(22) Date of filing: 29.08.2001
(51) Int. Cl.: A61K 47/12, A61K 47/32, A61P 5/24, A61K 31/568

(54) **Method of increasing testosterone and related steroid concentrations in women**

(30) Priority: 30.08.2000 US 651777; 01.11.2000 US 703753; 21.05.2001 US 292398 P
(62) Divisional of application: 01964525.8
(71) Applicant: Unimed Pharmaceuticals, LLC, Marietta, GA 30062 (US); LABORATOIRES BESINS INTERNATIONAL, 75003 Paris (FR)
(72) Inventor: Dudley, Robert E., Florida, il 32461 (US)
(74) Representative: Cabinet Plasseraud

(57) **Abstract**

The present invention relates to methods, kits, combinations, and compositions for treating, preventing or reducing the risk of developing a testosterone deficient disorder, or the symptoms associated with, or related to a testosterone deficient disorder in a female mammal in need thereof. The present invention also relates to a method of administering a steroid in the testosterone synthetic pathway, for example testosterone, to a mammal in need thereof. In addition, the methods, kits, combinations and compositions may be used in conjunction with other pharmaceutical agents effective at treating, preventing, or reducing the risk of developing a testosterone deficient disorder. The methods, kits, combinations and compositions can also be used in conjunction with a pharmacologically effective amount of an estrogenic hormone, for example, estradiol. Furthermore, the methods, kits, combinations and compositions can be used in conjunction with a pharmacologically effective amount of another steroid or pharmaceutical agent that increases serum testosterone levels in a mammal, for example, methyltestosterone.

## Description

### FIELD OF THE INVENTION

The present invention is related to methods, kits, combinations, and compositions for transdermally delivering an effective amount of testosterone using a hydroalcoholic gel formulation.

### DESCRIPTION OF THE RELATED ART

Transdermal preparations of testosterone have provided a useful delivery system for normalizing serum testosterone levels in hypogonadal men and preventing the clinical symptoms and long term effects of androgen deficient men. Available transdermal preparations of testosterone include, for example, TESTODERM®, TESTODERM® TTS, and ANDRODERM®. Testosterone is also available in other formulations including those available as an injectable, for example, DEPO-TESTOSTERONE® (testosterone cypionate), and DELATESTRYL BTG® (testosterone enanthate), or as a gel, for example, ANDROGEL® marketed by Unimed Pharmaceuticals, Inc., Deerfield, Illinois, the assignee of this application.

In men, transdermal patches are applied to the scrotal skin or other parts of the body. Recently, a one-percent testosterone gel has been approved for use in men, and provides dosing flexibility with minimal skin irritation. This gel is marketed under the name ANDROGEL®. However, all currently available testosterone transdermal products are specifically contraindicated for use in women in the United States. Furthermore, none of the currently available androgen treatment modalities for women, for example, oral methyltestosterone, intramuscular testosterone ester injections or subcutaneous testosterone implants can achieve reproducible testosterone serum levels on a consistent daily basis.

### A. Testosterone Physiology in Women

The excretion of androgenic steroids in the urine of adult women was demonstrated more than 50 years ago. Since that time, physiologists and clinicians have explored the sources and biological functions of testosterone and other endogenous androgenic hormones in the human female, see, for example, Geist S.H., Androgen therapy in the human female, J. Clin. Endocrinol. 1941; 1:154-161. It is now known that androgens are secreted by both the ovaries and adrenal glands in women. Each source contributes about 50% (directly and through precursors) (see, for example, Abraham G.E., Ovarian and adrenal contribution to peripheral androgens during the menstrual cycle, J. Clin. Endocrinol. Metab. 1974; 39:340-346) to the approximately 300 µg of testosterone produced daily in healthy "cycling" women (see, for example, Southren A. L., et al., Further study of factors affecting the metabolic clearance rate of testosterone in man, J. Clin. Endocrinol. Metab. 1968; 28:1105-1112). While the adverse effects of excess androgen production, as occurs in the polycystic ovary syndrome and certain androgen producing tumors, have been well described (see, for example, Lobo R.A., Chapter 20: Androgen excess in Infertility, Contraception and Reproductive Endocrinology, Third Edition. DR Mishell, V. Davajan and R. Lobo, Editors. Blackwell Scientific Publications, Boston. pp 422-446,1991), the normal physiological effects of androgens in women have been much less appreciated. As inferred from animal studies, male physiology, and the symptoms of women with deficient androgen production, the major physiological effects of androgens in normal women include, but are not limited to anabolic effects on muscle, skin, hair and bone; stimulatory effects on erythropoiesis; modulatory effects on immune function; and psychological effects on mood, well-being and sexual function.

In addition, endogenous androgens are important for the development of pubic hair and are thought to modulate the action of estrogens and progestins on a variety of reproductive target tissues. It is also believed that androgens play an important role in modulating the secretory function of the lacrimal gland.

Fifty percent of circulating testosterone is derived from direct ovarian secretion in the thecal cells under the control of luteinizing hormone. The other half is derived from peripheral conversion of adrenal androgen precursors dehydroepiandrosterone, androstenedione, and dehydroepiandrosterone sulfate. Testosterone can also be converted to dihydrotestosterone or estradiol. Thus, testosterone serves as both a hormone and as a prohormone.

Testosterone circulates in the blood 98% bound to protein. In women, approximately 66% of the binding is to the high-affinity sex hormone binding globulin. The remaining 34% is bound weakly to albumin. Thus, a number of measurements for testosterone are available from clinical laboratories. The term "free" testosterone as used herein refers to the fraction of testosterone in the blood that is not bound to protein. The term "total testosterone" or "testosterone" as used herein means the free testosterone plus protein-bound testosterone. The term "bioavailable testosterone" as used herein refers to the non-sex hormone binding globulin bound testosterone and includes that weakly bound to albumin. The order of affinity for the steroids most strongly bound by sex hormone binding globulin is dihydrotestosterone > testosterone > androstenedione > estrogen. Sex hormone binding globulin weakly binds dihydrotestosterone, but not dihydrotestosterone sulfate. Table 1 shows the approximate hormonal levels in normal pre-menopausal women.

**Table 1: Hormone Levels in Normal Pre-Menopausal Women**

| **Hormone** | **Mean ± sd** | **Median** | **Range** |
|---|---|---|---|
| **Testosterone (nmol/L)** | 1.20 ± 0.69 | 0.98 | 0.4 - 2.7 |
| **Free testosterone (pmol/L)** | 12.80 ± 5.59 | 12.53 | 4.1 - 24.2 |
| **% Free testosterone of total testosterone** | 1.4 ± 1.1 | 1.1 | 0.4 - 6.3 |
| **Luteinizing hormone (IU/L)** | 7.2 ± 3.3 | 6.7 | 3.0 - 18.7 |
| **Follicle stimulating hormone (IU/L)** | 4.7 ± 3.6 | 4.2 | 1.5 - 21.4 |
| **Sex hormone binding globulin (nmol/L)** | 66.1 ± 22.7 | 71.0 | 17.8 - 114.0 |

However, there is no general consensus on what constitutes "testosterone deficiency" in women because historically it has been impossible to develop assays capable of measuring such small hormonal levels. This is especially true when measuring free or bioavailable testosterone levels. Consequently, currently available laboratory evaluations, including measuring total, free, and bioavailable serum testosterone levels, have not been used extensively to identify hypoandrogenic women.

### B. Androgen Administration in Women

In comparison to other hormone deficiency states, testosterone deficiency in women has been largely ignored as a clinical entity. Nevertheless, there exist well-defined patient populations where androgen production is clearly deficient and where associated symptomatology has been described, including, for example, young oophorectomized/hysterectomized women, post-menopausal women on estrogen replacement therapy, women on oral contraceptives, women with adrenal dysfunction, women with corticosteroid-induced adrenal suppression, and human immunodeficiency virus-positive women.

Despite the clear benefits of administering testosterone to both normal and testosterone deficient women, almost all of the testosterone delivery preparations for human use are designed for hypogonadal men who require significantly greater amounts of testosterone than a testosterone deficient women. As a result, these formulations and devices are unsuitable for women requiring low doses of testosterone. Intramuscular injunction of testosterone esters, for example, is the popular form of androgen replacement for men but is unsatisfactory for women because of the very high levels of testosterone in the first 2-3 days after injection. Moreover, many women report increased acne and occasional cliteromegaly with this type of testosterone administration. Patients receiving injection therapy often complain that the delivery mechanism is painful and causes local skin reactions.

Because increasing testosterone concentrations has been shown to alter sexual performance and libido, researchers have investigated methods of delivering testosterone to men. These methods include intramuscular injections (43%), oral replacement (24%), pellet implants (23%), and transdermal patches (10%). A summary of these methods is shown in Table 2.

| **Table 2: Mode of Application and Dosage of Various Testosterone Preparations** | | |
|---|---|---|
| **Preparation** | **Route Of Application** | **Full Substitution Dose** |
| **In Clinical Use** | | |
| Testosterone enanthate | Intramuscular injection | 200-25.0 g every 2-3 weeks |
| Testosterone cypionate | Intramuscular injection | 200 mg every 2 weeks |
| Testosterone undecanoate | Oral | 2-4 capsules at 40 mg per day |
| Transdermal testosterone | Scrotal skin | 1 membrane per day |
| patch | Non-scrotal skin | 1 or 2 systems per day |
| Transdermal testosterone | Implantation under the | 3-6 implants of 200 mg every |
| patch | abdominal skin | 6 months |
| Testosterone implants | | |

| **Under Development** | | |
|---|---|---|
| Testosterone cyclodextrin | Sublingual | 2.5-5.0 mg twice daily |
| Testosterone undecanoate | Intramuscular injection | 1000 mg every 8-10 weeks |
| Testosterone buciclate | Intramuscular injection | 1000 mg every 12-16 weeks |
| Testosterone microspheres | Intramuscular injection | 315 mg for 11 weeks |

| **Obsolete** | | |
|---|---|---|
| 17∝-Methyltestosterone | Oral | 25-5.0 g per day |
| Fluoxymesterone | Sublingual | 10-25 mg per day |
| | Oral | 10-20 mg per day |

However, none of the current testosterone replacement products available for use in women are approved in the United States for chronic treatment of the female testosterone deficiency states described herein. Also, currently available methyltestosterone products, which can be administered orally, are no longer recommended as a testosterone replacement method for hypogonadal men, see, for example, GoorenLJ. G. and Polderman K. H., Safety aspects of androgens. In Testosterone: Action, Deficiency, Substitution. E. Nieschlag and HM. Behre, editors, Springer-Verlag, Heidelberg, p. 136 (1990). The long acting injectable testosterone-esters, such as enanthate or cypionate are formulated for high dose administration to men (for example 200 -300 mg) and produce supra-physiological hormone levels, even when given at lower doses to women (for example 50-100 mg) (see, for example, Sherwin B.B. and Gelfand M.M., Differential symptom response to parenteral estrogen and/or androgen administration in the surgical menopause, Am. J. Obstet. Gynecol. 1985; 151:153-160). Testosterone implants, which have been used experimentally in the past, can likewise produce supra-physiological hormone levels in women, see, for example, Burger H.G. et al., The management of persistent menopausal symptoms with oestradioltestosterone implants: clinical, lipid and hormonal results, Maturitas 1984; 6:351-358. The supra-physiological androgen levels associated with these products have produced virilizing side effects in some patients, see for example, Burger H.G. et al., (1984). Also see, for example, Sherwin B.B, and Gelfand M. M., (1985). Also see, for example, Urman B., et al., Elevated serum testosterone, hirsutism and virilism associated with combined androgen-estrogen hormone replacement therapy, Obstet. Gynecol., 1991; 7:595-598.

Given the above, however, ESTRATEST®, which is a combination of methyltestosterone and esterified estrogens in oral tablet formulations, is the most commonly used androgen product used to treat women in the United States. At present, however, its only approved indication is for the treatment of moderate to severe vasomotor symptoms associated with menopause in those patients not improved by estrogens alone. Pharmacological doses of methyltestosterone higher than those suggested for hypogonadal men have also been used to treat breast cancer in women. However, oral administration produces inappropriate testosterone levels and unpredictable absorption patters between patients (Buckler 1998). Moreover, because the liver metabolizes the preparation, there is a risk ofhepatoxicity not to mention first pass metabolism.

Testosterone pellet implants (50 mg or 100 mg of testosterone) inserted under local anesthesia in the abdominal wall have been used in conjunction with estrogen pellet implants for many years. Testosterone levels peak about one month after implantation and then return to baseline by month five or six. The testosterone levels are high and characterized by substantial rises and falls over several months and marked individual variation in this period. In addition, implants require a surgical procedure that many men and women simply do not wish to endure. In hypogonadal men, for example, implant therapy includes a risk of extrusion (8.5%), bleeding (2.3%), or infection (0.6%).

Given the problems associated with injected, orally administered and implant-based testosterone delivery methods, researchers have recently begun experimenting with more controlled release preparations that can deliver stable and physiological testosterone levels to women. In the past decade, the transdermal delivery of estradiol has become recognized as a safe, physiological and patient-friendly method for estrogen replacement therapy in women. Second generation estradiol patches that use adhesive matrix technology have recently become available in the United States and Europe. Matrix technology now exists to transdermally administer physiological amounts of testosterone alone for the treatment of androgen deficiency states in women. As the patient populations defined above are approximately 50% deficient in their testosterone production, the transdermal systems have been designed to deliver approximately half of the normal daily testosterone production rate or about 150 µg per day. Matrix technology-based transdermal testosterone administration has been used successfully in women to treat acquired immunodeficiency syndrome wasting and female sexual dysfunction after oophorectomy.

Two testosterone patches for women have been tested in clinical studies. Buckler and his associates have investigated a testosterone patch (Ethical Pharmaceuticals, UK) delivering either 840, 1100, 3000 µg testosterone per day applied twice weekly to the anterior abdominal wall, but did not disclose the composition of the patch (Buckler 1998). Another patch, the TMTDS patch (Watson Laboratories, Salt Lake City, UT), is a translucent patch having a surface area of 18 cm² which uses sorbitan monooleate as a permeation enhancer and a hypoallergenic acrylic adhesive in an alcohol-free matrix. The average testosterone content of each patch is 4.1 mg. Each patch is designed to deliver testosterone at a nominal rate of 150 g of testosterone per day over an application period of three to four days. Thus, the TMTDS patch is applied twice per week (Javanbakht et al. 2000).

While clinical studies have reported that the testosterone-containing patch is capable of increasing testosterone concentrations in women via a controlled release mechanism, the patches do not provide dosing flexibility. Moreover, their visibility may be esthetically unappealing to some women and may have a tendency to fall off, especially during rigorous physical exercise.

For these and other reasons, therefore, it would be a difficult but much desired advance in the art to provide an effective percutaneously administered testosterone formulation to be applied directly to the skin of a women in the form of, for example, a gel, ointment, or cream, to treat testosterone deficient disorders.

### DETAILED DESCRIPTION OF THE PRESENT INVENTION

While the present invention may be embodied in many different forms, several specific embodiments are discussed herein with the understanding that the present disclosure is to be considered only as an exemplification of the principles of the invention, and it is not intended to limit the invention to the embodiments illustrated.

Where the invention is illustrated herein with particular reference to testosterone, it will be understood that any other steroid in the testosterone synthetic pathway can, if desired, be substituted in whole or in part for testosterone in the methods, kits, combinations, and compositions herein described. Where the invention is illustrated herein with particular reference to methyltestosterone, it will be understood that any other inhibitor of the synthesis of sex hormone binding globulin can, if desired, be substituted in whole or in part for methyltestosterone in the methods, kits, combinations, and compositions herein described. Where the invention is illustrated herein with particular reference to estradiol, it will be understood that any other estrogenic hormone can, if desired, be substituted in whole or in part for estradiol in the methods, kits, combinations, and compositions herein described.

The present invention is directed to methods, kits, combinations, and compositions for treating, preventing or reducing the risk of developing a testosterone deficient disorder, or the symptoms associated with, or related to a testosterone deficient disorder in a female mammal in need thereof. The method comprises percutaneously administering a testosterone deficient disorder-effective amount of steroid in the testosterone synthetic pathway, for example, testosterone, to a female mammal. The present invention includes methods of reversing, halting or slowing the progression of a testosterone deficient disorder once it becomes clinically evident, or treating the symptoms associated with, or related to the testosterone deficient disorder. The patient may already have a testosterone deficient disorder at the time of administration, or be at risk of developing a testosterone deficient disorder. Also included in the present invention is a method of administering a steroid in the testosterone synthetic pathway, for example testosterone, to a mammal in need thereof. The method comprises administering to the mammal a testosterone deficient disorder-effective amount of a percutaneously deliverable composition comprised of a pharmaceutically-acceptable steroid in the testosterone synthetic pathway, for example testosterone, one or more lower alcohols, such as ethanol or isopropanol, a penetration enhancing agent, a thickener, and water. Also included in the methods, kits, combinations, and compositions of the present invention are pharmaceutical compositions comprising a testosterone deficient disorder-effective amount of testosterone. In one embodiment the testosterone composition is formulated as a hydroalcoholic gel. In another embodiment, the gel comprises testosterone, one or more lower alcohols, such as ethanol or isopropanol, a penetration enhancing agent, a thickener, and water. The present invention also includes kits comprising percutaneously deliverable testosterone. The kits also contain a set of instructions for the patient. In another embodiment, the methods, kits, combinations, and compositions are used in conjunction with other steroids or pharmaceutical agents effective at treating, preventing, or reducing the risk of developing a testosterone deficient disorder. In one embodiment, the present invention employing testosterone is used in conjunction with a pharmacologically effective amount of an estrogenic hormone, for example, estradiol either in the same dosage form or as separate dosage forms. In another embodiment, the methods, kits, combinations, and compositions are used with another steroid or pharmaceutical agent that increases testosterone levels in a mammal, for example, methyltestosterone. Additionally, the present invention optionally include salts, esters, amides, enantiomers, isomers, tautomers, prodrugs, or derivatives of the compounds of the present invention, as well as emollients, stabilizers, antimicrobials, fragrances, and propellants. The methods, kits, combinations, and compositions of the present invention provide enhanced treatment options for treating a testosterone deficient disorder in a female mammal, for example, a women, as compared to those currently available.

Besides being useful for human treatment, the present invention is also useful for veterinary treatment of companion mammals, exotic animals and farm animals, including mammals, rodents, and the like. In one embodiment, the mammals include horses, dogs, and cats.

A class of steroids in the testosterone synthetic pathway useful in the methods, kits, combinations, and compositions of the present invention include steroids in the testosterone anabolic or catabolic pathway. In a broad aspect of the invention, the active ingredients employed in the composition may include anabolic steroids such as androisoxazole, bolasterone, clostebol, ethylestrenol, formyldienolone, 4-hydroxy-19-nortestosterone, methenolone, methyltrienolone, nandrolone, oxymesterone, quinbolone, stenbolone, trenbolone; androgenic steroids such as boldenone, fluoxymesterone, mestanolone, mesterolone, methandrostenolone, 17 ∝ methyltestosterone, 17 alpha-methyl-testosterone 3-cyclopentyl enol ether, norethandrolone, normethandrone, oxandrolone, oxymetholone, prasterone, stanlolone, stanozolol, dihydrotestosterone, testosterone; and progestogens such as anagestone, chlormadinone acetate, delmadinone acetate, demegestone, dimethisterone, dihydrogesterone, ethinylestrenol, ethisterone, ethynodiol, ethynodiol diacetate, flurogestone acetate, gestodene, gestonorone caproate, haloprogesterone, 17-hydroxy-16-methylene-progesterone, 17 alpha-hydroxyprogesterone, 17 alpha-hydroxyprogesterone caproate, medrogestone, medroxyprogesterone, megestrol acetate, melengestrol, norethindrone, norethindrone acetate, norethynodrel, norgesterone, norgestimate, norgestrel, norgestrienone, 19-norprogesterone, norvinisterone, pentagestrone, progesterone, promegestone, quingestrone, and trengestone; and all salts, esters, amides, enantiomers, isomers, tautomers, prodrugs and derivatives of these compounds. (Based upon the list provided in The Merck Index, Merck & Co. Rahway, N.J. (1998)). Combinations of the above mentioned steroids can be used.

In one embodiment, testosterone is formulated as a hydroalcoholic gel. In another embodiment, the gel comprises testosterone, one or more lower alcohols, such as ethanol or isopropanol, a penetration enhancing agent, a thickener, and water. Additionally, the gel optionally includes the salts, esters, amides, enantiomers, isomers, tautomers, prodrugs, or derivatives of testosterone, as well as emollients, stabilizers, antimicrobials, fragrances, and propellants.

Illustratively, certain formulations of the present invention deliver about 0.01 g to about 100.0 g testosterone, or the equivalent thereof, to a patient per dosage unit. In another embodiment of the present invention, the formulations deliver from about 0.1 g to about 10.0 g testosterone, or the equivalent thereof, to a patient per dosage unit. In yet another embodiment of the present invention, the formulations of the present invention deliver from about 0.17 g to about 0.5 g testosterone, or the equivalent thereof, to a patient per dosage unit. In still another embodiment of the present invention, the formulations of the present invention deliver about 0.25 g testosterone, or the equivalent thereof, to a patient per dosage unit. Thus, for example, a testosterone gel formulated as a single dosage unit for once a day administration contains about 0.17 g, or about 0.25 g, or about 0.5 g testosterone, while a gel formulated as a single dosage unit for once a week administration contains about 1.19 g, or about 1.75 g, or about 3.50 g testosterone, respectfully.

In one embodiment, the formulation is a gel and is comprised of the following substances in approximate amounts:

**Table 3: Composition of Testosterone Gel**

| **SUBSTANCE** | **AMOUNT (w/w)** **PER 100g OF** **GEL** |
|---|---|
| Testosterone | 1.0 g |
| Carbopol 980 | 0.90 g |
| Isopropyl myristate | 0.50 g |
| 0.1 N NaOH | 4.72 g |
| Ethanol (95% w/w) | 72.5 g* |
| Purified water (qsf) | 100 g |

| | |
|---|---|
| *Corresponding to 67 g of ethanol. | |

The gel is rubbed onto the clean dry skin of the upper outer thigh and hip once daily. Following application, the gel is allowed to air dry. The patient washes her hands. Application of the gel results in an increased testosterone level having a desirable pharmacokinetic profile similar to that in normal women. The gel is thus useful for treating a number of conditions or diseases in women.

Achieving target delivery rates demonstrated by testosterone gel can be estimated from the pharmacokinetics in testosterone gel in men. The mean serum concentration (Cavg) values in men after applying of varying amounts of gel to the upper body is given below in Table 4.

**Table 4 Mean Average Serum Testosterone Concentrations and Daily Delivery Rate after Administration of Testosterone Gel 1% in Men**

| Dose (µL) (gram) | Mean Cavg (ng/dL) | Daily Delivery Rate (µg/day)^{a} |
|---|---|---|
| 5.0 | 555 (± 225) | 3330 |
| 7.5 | 601 (± 309) | 3606 |
| 10.0 | 713 (± 209) | 4278 |

| | | |
|---|---|---|
| ^{a} Metabolic Clearance Rate of Daily Testosterone = 600 L/day | | |

Based on the results obtained in men, a testosterone gel dose of 0.5 grams delivers approximately 300 µg of testosterone per day.

Illustratively, for an adult woman, a testosterone deficient disorder-effective amount of testosterone per daily dose delivers to the blood serum typically about 100 µg to about 150 µg to about 300µg of testosterone per day. Thus, to achieve a serum blood level of about 100 µg testosterone, RELIBRA™ (applicant's trademark for gel product for women) is administered at about 0.17 g/day, which delivers about 1.7 mg/day of testosterone to the skin of which about 0.1 mg, is absorbed; or to achieve a serum blood level of about 150 µg testosterone, RELIBRA is administered at about 0.25 g/day, which delivers about 2.5 mg/day of testosterone to the skin of which about 0.15 mg, is absorbed; or to achieve a serum blood level of about 300 µg testosterone, RELIBRA is administered at about 0.5 g/day, which delivers 5.0 mg/day of testosterone to the skin of which about 0.3 mg, is absorbed.

One skilled in the art will appreciate that the constituents of this formulation may be varied in amounts yet continue to be within the spirit and scope of the present invention. For example, the composition may contain about 0.01 to about 100.0 g of testosterone, about 0.1 to about 5.0 g Carbopol, about 0.1 to about 5.0 g isopropyl myristate, and about 30.0 to about 98 g ethanol.

The use of the term "about" in the present disclosure means "approximately," and use of the term "about" indicates that dosages slightly outside the cited ranges may also be effective and safe, and such dosages are also encompassed by the scope of the present claims.

The phrase "pharmaceutically acceptable" is used adjectivally herein to mean that the modified noun is appropriate for use in a pharmaceutical product. Pharmaceutically acceptable cations include metallic ions and organic ions. More preferred metallic ions include, but are not limited to appropriate alkali metal salts, alkaline earth metal salts and other physiological acceptable metal ions. Exemplary ions include aluminum, calcium, lithium, magnesium, potassium, sodium and zinc in their usual valences. Preferred organic ions include protonated tertiary amines and quaternary ammonium cations, including in part, trimethylamine, diethylamine, N,N'-dibenzylethylenediamine, chloroprocaine, choline, diethanolamine, ethylenediamine, meglumine (N-methylglucamine) and procaine. Exemplary pharmaceutically acceptable acids include without limitation hydrochloric acid, hydrobromic acid, phosphoric acid, sulfuric acid, methanesulfonic acid, acetic acid, formic acid, tartaric acid, maleic acid, malic acid, citric acid, isocitric acid, succinic acid, lactic acid, gluconic acid, glucuronic acid, pyruvic acid oxalacetic acid, fumaric acid, propionic acid, aspartic acid, glutamic acid, benzoic acid, and the like.

The phrase "penetration enhancer" refers to an agent known to accelerate the delivery of the drug through the skin. These agents also have been referred to as accelerants, adjuvants, and absorption promoters, and are collectively referred to herein as "enhancers." This class of agents includes those with diverse mechanisms of action including those which have the function of improving the solubility and diffusibility of the drug, and those which improve percutaneous absorption by changing the ability of the stratum corneum to retain moisture, softening the skin, improving the skin's permeability, acting as penetration assistants or hair-follicle openers or changing the state of the skin such as the boundary layer. The penetration enhancer of the present invention is a functional derivative of a fatty acid, which includes isosteric modifications of fatty acids or non-acidic derivatives of the carboxylic functional group of a fatty acid or isosteric modifications thereof. In one embodiment, the functional derivative of a fatty acid is an unsaturated alkanoic acid in which the --COOH group is substituted with a functional derivative thereof, such as alcohols, polyols, amides and substituted derivatives thereof. The term "fatty acid" means a fatty acid that has four (4) to twenty-four (24) carbon atoms.

Non-limiting examples of penetration enhancers include C8-C22 fatty acids such as isostearic acid, octanoic acid, and oleic acid; C8-C22 fatty alcohols such as oleyl alcohol and lauryl alcohol; lower alkyl esters of C8-C22 fatty acids such as ethyl oleate, isopropyl myristate, butyl stearate, and methyl laurate; di(lower)alkyl esters of C6-C22 diacids such as diisopropyl adipate; monoglycerides of C8-C22 fatty acids such as glyceryl monolaurate; tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; N-alkylpyrrolidone; and terpenes.

The thickeners used herein may include anionic polymers such as polyacrylic acid (CARBOPOL® by B.F. Goodrich Specialty Polymers and Chemicals Division of Cleveland, Ohio), carboxymethylcellulose and the like. Additional thickeners, enhancers and adjuvants may generally be found in Remington's The Science and Practice of Pharmacy, Meade Publishing Co., United States Pharmaeopeia/National Formulary.

As used herein, the term "lower alcohol," alone or in combination, means a straight-chain or branched-chain alcohol moiety containing one to about six carbon atoms. In one embodiment, the lower alcohol contains one to about 4 carbon atoms, and in another embodiment the lower alcohol contains two to about 3 carbon atoms. Examples of such alcohol moieties include methanol, ethanol, n-propanol, isopropanol, n-butanol, isobutanol, sec-butanol, and tert-butanol.

As used herein, the term "lower alkyl", alone or in combination, means a straight-chain or branched-chain alkyl radical containing one to about six carbon atoms. In one embodiment, the lower alkyl contains one to about four carbon atoms. Examples of such radicals include methyl, ethyl, n-propyl, isopropyl, n-butyl, isobutyl, sec-butyl, and tert-butyl.

The term "treat" or "treatment" as used herein refers to any treatment of a mammalian condition, disorder, or disease associated with an androgen deficiency or a testosterone deficiency, and includes, but is not limited to, preventing the condition, disorder, or disease from occurring in a mammal which may be predisposed to the condition, disorder, or disease, but has not yet been diagnosed as having the condition, disorder, or disease; inhibiting the condition, disorder, or disease, for example, arresting the development of the condition, disorder, or disease; relieving the condition, disorder, or disease, for example, causing regression of the condition, disorder, or disease; or relieving the condition caused by the disease or disorder, for example, stopping the symptoms of the disease or disorder.

The term "prevent" or "prevention," in relation to a testosterone deficient condition, disorder, or disease, means no testosterone deficient condition, disorder, or disease development if none had occurred, or no further testosterone deficient condition, disorder, or disease development if there had already been development of the testosterone deficient condition, disorder, or disease.

The phrase "testosterone deficient disorder" refers to a to a condition, disorder, or disease that occurs in a mammal due to lack of endogenous testosterone production. In women, such conditions, disorders, or diseases include, but are not limited to, hypogonadism, sexual dysfunction, decreased libido, hypercholesterolemia, abnormal electrocardiograms, vasomotor symptoms, diabetic retinopathy, hyperglycemia, hyperinsulinemia, hypoinsulinemia, increased percentage of body fat, hypertension, obesity, osteoporosis, osteopenia, vaginal dryness, thinning of the vaginal wall, menopausal symptoms and hot flashes, cognitive dysfunction, cardiovascular disease, Alzheimer's disease, dementia, cataracts, and cervical cancer uterine cancer or breast cancer.

Decreased production of testosterone by a woman can be caused by several factors, including, but not limited to, use of oral contraceptives; surgery, for example, removal of the uterus (hysterectomy), or removal of one of both ovaries (oophorecty/ ovariectomy); estrogen replacement therapy in post-menopausal women; premature ovarian failure; adrenal dysfunction, for example primary adrenal insufficiency; coricosteroid-induced adrenal suppression; panhypopituitarism; and chronic illness, such as systemic lupus erythematosis, rheumatoid arthritis, human immunodeficiency virus (HIV) infection, chronic obstructive lung disease, and end stage renal disease.

Physiological and psychological disorders associated with testosterone deficiency in a woman include, for example, decreased libido and sexual performance, decreased bone mineral density and related markers, diminished body composition, human immunodeficiency virus wasting syndrome, decreased cognition, diminished mood and self-esteem, decreased muscle mass and performance, premenstrual syndrome, and autoimmune disease.

A "testosterone deficient disorder effect" or "testosterone deficient disorder-effective amount" is intended to qualify the amount of testosterone required to treat or prevent a testosterone deficient disorder in a mammal, or relieve to some extent one or more of the symptoms associated with, or related to, a testosterone deficient disorder in a mammal. In a woman, this includes, but is not limited to, normalizing hypogonadism; improving sexual dysfunction; increasing libido; normalizing cholesterol levels; normalizing abnormal electrocardiograms of patients and improving vasomotor symptoms; improving diabetic retinopathy as well as lowering the insulin requirements of diabetic patients; decreasing the percentage of body fat; normalizing glucose levels; decreasing the risk factors for cardiovascular disease, including normalizing hypertension, and treating obesity; preventing osteoporosis, osteopenia, vaginal dryness, and thinning of the vaginal wall; relieving menopausal symptoms and hot flashes; improving cognitive dysfunction; treating, preventing or reducing the onset of cardiovascular disease, Alzheimer's disease, dementia, and cataracts; and treating, preventing or reducing the risk of cervical, uterine or breast cancer.

The compositions of the present invention are used in a "testosterone deficient disorder effective amount." This means that the concentration of the testosterone is such that a therapeutic level of drug is delivered over the term that the percutaneously delivered formulation is to be used. Such delivery is dependent on a number of variables including the time period for which the individual dosage unit is to be used, the flux rate of the therapeutic agent, for example, testosterone, from the gel, surface area of application site, etc. The amount of therapeutic agent necessary can be experimentally determined based on the flux rate of the drug through the gel, and through the skin when used with and without enhancers. It is understood, however, that specific dose levels of the therapeutic agents of the present invention for any particular patient depends upon a variety of factors including the activity of the specific compound employed, the age, body weight, general health, sex, and diet of the patient, the time of administration, the rate of excretion, the drug combination, and the severity of the particular disorder being treated and form of administration. Treatment dosages generally may be titrated to optimize safety and efficacy. Typically, dosage-effect relationships from *in vitro* and/or *in vivo* tests initially can provide useful guidance on the proper doses for patient administration. Studies in animal models generally may be used for guidance regarding effective dosages for treatment of menopause in accordance with the present invention. In terms of treatment protocols, it should be appreciated that the dosage to be administered will depend on several factors, including the particular agent that is administered, the route administered the condition of the particular patient, etc. Generally speaking, one will desire to administer an amount of the compound that is effective to achieve a serum level commensurate with the concentrations found to be effective *in vitro.* Thus, where an compound is found to demonstrate *in vitro* activity at, for example, 10 ng/ml, one will desire to administer an amount of the drug that is effective to provide about a 10 ng/ml concentration *in vivo.* Determination of these parameters is well within the skill of the art. These considerations, as well as effective formulations and administration,procedures are well known in the art and are described in standard textbooks.

In order to measure and determine the testosterone deficient-effective amount of testosterone to be delivered to a subject, serum testosterone concentrations can be measured using standard assay techniques. Free serum testosterone levels are measured by the recently validated and highly sensitive equilibrium dialysis method discussed in Sinha-Hikim et al., The Use of a Sensitive Equilibrium Dialysis Method for the Measurement of Free Testosterone Levels in Healthy, Cycling Women and in HIV-Infected Women, 83 J. CLINICAL ENDOCRINOLOGY & METABOLISM 1312-18. (1998), and is herein fully incorporated by reference.

As used herein, the phrases "androgen deficiency" or "testosterone deficiency" are used interchangeably, and refer to lower serum levels of free testosterone in a subject as compared to the median serum levels for healthy women of the same age. Normal cycling women produce approximately 300 µg of testosterone per day. Their total serum testosterone levels generally range from about 20 ng/dL to about 80 ng/dL averaging about 40 ng/dL. In healthy young women, for example, mean free testosterone levels are generally about 3.6 pg/mL. However, several factors may influence both total and free testosterone serum levels. For example, in regularly ovulating women, there is a small but significant increase in plasma testosterone levels during the middle third of the menstrual cycle. However, mean testosterone levels (1.2 nmol/L or 33 ng/dL) and mean free testosterone levels (12.8 pmol/L or 3.6 pg/mL) during the luteal and follicular phases are not significantly different. Additionally, testosterone production declines continuously after age 30 so that serum testosterone levels in a 60-year-old woman are only 50% of the levels in a young 30-year-old woman. Although the percentage of free testosterone generally does not vary with age, an absolute decline in free testosterone has been observed. This decline does not occur abruptly at menopause but instead occurs gradually and continuously as a result of the age-related decrease in both the adrenal and ovarian androgen production. Thus, women begin to experience symptoms associated with menopause in the immediate pre-menopausal years. The decline in testosterone following menopause results from the combination of ovarian failure, decreasing renal secretion, and peripheral conversion. Also, for example, after ovariectomy, testosterone concentrations decrease by about 50%. Diagnosis of a testosterone deficiency is known to the average physician practicing in the relevant field of medicine.

Nevertheless, there exist well-defined patient populations where testosterone production is clearly deficient and where associated symptomatology has been described, and such populations are contemplated as falling within the scope of the present invention.

Patients to be treated with the present invention include those at risk of developing a testosterone deficient disorder, or patients currently experiencing a testosterone deficient disorder event. Standard testosterone deficient disorder risk factors are known to the average physician practicing in the relevant field of medicine. Patients who are identified as having one or more risk factors known in the art to be at risk of developing a testosterone deficient disorder, as well as people who already have a testosterone deficient disorder, are intended to be included within the group of people considered to be at risk for having a testosterone deficient disorder event.

In addition, contemplated methods, kits, combinations, and compositions of the present invention are useful to treat testosterone deficiency in a woman, which includes a woman where testosterone production is deficient, or where the associated symptomatology related to deficient testosterone production is clinically evident. This includes, for example, a oophorectomized/hysterectomized woman, a post-menopausal woman on estrogen replacement therapy, a woman on oral contraceptives, a woman with an ovariectomy, a woman with premature ovarian failure, a woman with adrenal dysfunction, a woman with corticosteroid-induced adrenal suppression, a woman with panhypopituitarism, a woman with primary adrenal insufficiency, and a woman experiencing chronic illness, such as systemic lupus erythematosis, rheumatoid arthritis, human immunodeficiency virus (HIV) infection, chronic obstructive lung disease, and end stage renal disease.

In one embodiment of the present invention, the methods, kits, combinations, and composition are useful in treating a woman who have undergone surgery, including, for example, bilateral oophorectomy with hysterectomy, and particularly a woman whose surgery was performed at a younger age, prior to her natural menopause. In the U.S. alone, more than 250,000 women undergo combined oophorectomy/hysterectomy procedures annually and are clearly deficient in testosterone production. Serum testosterone levels typically decrease by 50% in a oophorectomized woman compared to their pre-operative levels, however, in some cases the levels may still remain within the normal reference range (approximately 20 - 80 ng/dL). Estrogen and progesterone levels, which are primarily dependent on ovarian secretion, are also markedly reduced after oophorectomy. The resulting multiple hormone deficiency state is associated with vasomotor symptoms, high-turnover osteoporosis, and female sexual dysfunction. While estrogen replacement therapy is standard for the treatment of vasomotor symptoms and osteoporosis in the oophorectomized/hysterectomized female, concomitant testosterone therapy has not been indicated for treatment of female sexual dysfunction or for its effects with estrogen replacement therapy on bone metabolism. Such women are contemplated as falling within the scope of the present invention.

In another embodiment of the present invention, the methods, kits, combinations, and composition are useful in treating a post-menopausal woman. In contrast to the oophorectomized state, the post-menopausal ovary may continue to synthesize testosterones in the stromal tissue at rates that are not necessarily lower than the premenopausal period. In some post-menopausal women, testosterone levels increase as a consequence of the stromal response to elevated luteinizing hormone levels, while in others testosterone levels decrease or remain the same. Since estrogen replacement therapy lowers luteinizing hormone levels, ovarian testosterone secretion would be expected to decrease in post-menopausal women who receive estrogen replacement therapy. With oral estrogen replacement therapy preparations, the fall in testosterone levels may be obscured by the concomitant rise in sex hormone binding globulin levels, which reduces testosterone clearance. However, free and/or bioavailable testosterone levels are found to be lower in a post-menopausal woman receiving oral estrogen replacement therapy. While the effects of transdermal estrogen replacement therapy on the androgen/luteinizing hormone status of post-menopausal women has not been studied, a reduction in total and free testosterone levels, associated with a decrease in luteinizing hormone levels, would also be expected. As many post-menopausal women experience symptoms of female sexual dysfunction that are not ameliorated by estrogen replacement therapy, it is believed that testosterone deficiency is a contributing factor, and this group of women would fall within the scope of the present invention.

In yet another embodiment of the present invention, the methods, kits, combinations, and composition are useful in treating a woman who uses oral contraception. Oral contraception is the most common method of contraception among adolescents, and overall about 46% of the sexually active population use oral contraception. The most common type of oral contraceptive contains both estrogen and progestin and has proven to be about 99% effective. Thus, almost half of all premenopausal women (<44 years old) are potentially taking oral contraceptives. In comparison to healthy "cycling" women, the testosterone levels in women treated with estrogen-containing oral contraceptives are markedly lower, particularly when compared at the pre-ovulatory phase of the normal cycle, when testosterone levels are highest. This effect result from the luteinizing hormone suppression produced by oral contraceptives and is analogous to the effect of estrogen replacement therapy described above. Psychosexual aspects of perception are affected by the lower testosterone levels and may be related to the clinical observation of decreased libido in some women using oral contraceptives.

In yet another embodiment of the present invention, the methods, kits, combinations; and composition are useful in treating a woman who have an undergone an ovariectomy by, for example, surgery, chemical means, irradiation, or gonadotropin-releasing hormone antagonists. Such surgery leads to decreased ovarian androgen product.

In another embodiment of the present invention, the methods, kits, combinations, and composition are useful in treating a woman with premature ovarian failure. Premature ovarian failure, such as that associated with Turner's Syndrome or the autoimmune or idiopathic destruction of the ovary, is associated with impaired testosterone production.

In still another embodiment of the present invention, the methods, kits, combinations, and composition are useful in treating a woman who has decreased adrenal function. Decrease adrenal function, which may result from a variety of causes, represents another category of patients where testosterone production may be reduced by approximately 50%. Primary adrenocortical deficiency, or Addison's disease, is a rare endocrine disorder with multiple etiologies, including tuberculosis and fungal infections. The estimated prevalence in women is approximately 5 per 100,000. Due to the lack of gluco- and mineral corticoid secretion, Addison's disease can be life threatening. While some researchers have noted the associated testosterone deficiency, replacement therapy is often ignored. As the adrenocorticotropic hormone appears to be the primary stimulator of adrenal androgen production, deficient adrenocorticotropic hormone secretion can also lead to testosterone deficiency in women. This can result from pituitary disease or surgery, for example, secondary adrenocortical deficiency, or as a pharmacological effect of exogenous corticosteroid administration that can suppress adrenocorticotropic hormone secretion.

In one embodiment of the present invention, the methods, kits, combinations, and composition are useful in treating a woman where chronic corticosteroid therapy is administered. Chronic corticosteroid therapy is used for a variety of conditions, which include rheumatoid arthritis, systemic lupus erythematosus, Sjogren's syndrome, immunosuppression for transplants, asthma, etc. Corticosteroid-induced adrenal suppression may thus represent the largest group of patients with deficient adrenal androgen production. Androgen deficiency is recognized as a contributory factor to corticosteroid-induced osteoporosis. By stimulating bone formation (osteoblast activity), testosterone replacement is beneficial in the treatment of corticosteroid-induced osteoporosis in premenopausal women, and is beneficial in estrogen replacement therapy where treating post-menopausal women. In a woman with autoimmune disorders, such as rheumatoid arthritis and systemic lupus erythematosus, testosterone deficiency can contribute to the underlying tendency to produce autoantibodies, as has been seen in a variety of animal models of autoimmune disease. Testosterone replacement can thus help to ameliorate the autoimmune disease process, itself. Despite these considerations, the potential therapeutic benefits of testosterone replacement in treating corticosteroid suppressed women have largely been ignored.

In another embodiment of the present invention, the methods, kits, combinations, and composition are useful in treating a panhypopituitarism woman. Panhypopituitarism from any cause is attended by a severe testosterone deficiency because of derangement of androgen secretion by both the ovaries and the adrenal glands.

In yet another embodiment of the present invention, the methods, kits, combinations, and composition are useful in treating a woman with primary adrenal insufficiency. Primary adrenal insufficiency is associated with testosterone deficiency.

In one embodiment of the present invention, the methods, kits, combinations, and composition are useful in treating a woman with chronic illnesses. Chronic illnesses in a woman are attended by decreased circulating testosterone concentrations. Glucocorticoid administration inhibits adrenal androgen production by their inhibitory effects on adrenocorticotropic hormone secretion. In addition, glucocorticoids also have inhibitory effects at all levels of the hypothalamic-pituitary-ovarian axis.

In still another embodiment of the present invention, the methods, kits, combinations, and composition are useful in treating a human immunodeficiency virus-positive woman. In contrast to human immunodeficiency virus-positive men, where testosterone deficiency is common, it is not known whether human immunodeficiency virus-positive women are deficient in testosterone. Amenorrhea, which appears to be increased in women with acquired immunodeficiency syndrome (AIDS), may be an indication that ovarian steroid production is diminished. Adrenal function can also be deficient in acquired immunodeficiency syndrome patients due to cytomegalovirus infection, tuberculosis and/or fungal infections. Megestrol acetate, a progestational agent used to stimulate appetite in human immunodeficiency virus infected persons, suppresses gonadotropins and is it believed to lower testosterone levels in women, similar to its effects in men. In addition, the use of oral contraceptives by a human immunodeficiency virus-positive woman also reduces testosterone levels, as described above in normal women. Physiological testosterone replacement can be used as an anabolic agent for treating/preventing the wasting syndrome and for enhancing quality of life in a woman.

The methods, kits, combinations, and compositions of the present invention are also useful to treat a number of physiological and psychological parameters associated with testosterone deficiency in a woman, and include, for example, increasing libido and improving sexual performance and dysfuntion, increasing bone mineral density and related markers, improving body composition, preventing human immunodeficiency virus wasting syndrome, improving cognition, improving mood and self-esteem, improving muscle mass and performance, treating premenstrual syndrome, and treating autoimmune diseases.

In one embodiment of the present invention, the methods, kits, combinations, and composition are useful in treating the libido of a woman. Testosterone concentrations clearly affect female libido. Over the past few decades, several correlational studies found that higher testosterone levels were associated with less sexual avoidance, more sexual gratification, more sexual thoughts, more initiation of sexual activity, higher levels of sexual interest and desire, and more anticipation of sexual activity. More recently, found a correlation between sexual desire and testosterone in a subset of women, those who were human immunodeficiency virus-positive.

In one embodiment of the present invention, the methods, kits, combinations, and composition are useful in treating sexual performance in a woman. Studies have shown that testosterone influences sexual performance in women. Correlational studies have found that testosterone is associated with higher sexual arousability as measured by vasocongestive responses to erotic films, increased frequency of masturbation, increased frequency of coitus, and a higher number of sexual partners. Another correlational study also showed that testosterone is associated with decreased vaginal atrophy.

In another embodiment of the present invention, the methods, kits, combinations, and composition are useful in treating female sexual dysfunction in a woman. Surgical menopause, that is, total abdominal hysterectomy and bilateral salpingo-oophorectomy, performed prior to the natural menopause causes a syndrome of female sexual dysfunction in a significant number of women that is unrelieved by conventional estrogen replacement therapy. The sexual components of this syndrome include decreased libido, decreased arousal and a diminished ability to attain orgasm. The psychological components include decreased energy, depressed mood, and a general decrease in well-being. These are generally distinguishable from the classic estrogen deficiency symptoms of vaginal atrophy, diminished lubrication, hot flushes and emotional liability that can adversely affect sexual function and psychological well-being in menopausal women who do not receive adequate estrogen replacement therapy. Rather than estrogen deficiency, the hormonal basis for this syndrome is attributed to a testosterone deficiency state resulting from the absent ovarian production of testosterone and its precursors.

In one study, the effects of testosterone in women with impaired sexual function after surgically induced menopause were evaluated using a transdermal patch. Seventy-five women, 31 to 56 years old, who had undergone oophorectomy and hysterectomy received conjugated equine estrogens (at least 0.625 mg per day orally) and, in random order, 150 µg of testosterone, and 300 µg of testosterone per day transdermally for 12 weeks each. Outcome measures included scores on the Brief Index of Sexual Functioning for Women (BISF), the Psychological Well-Being Index (PGWI), and a sexual function diary completed over the telephone. The mean (±SD) serum free testosterone concentration increased from 1.2 ± 0.8 pg/mL during placebo treatment to 3.9 ± 2.4 pg/mL and 4.9 ± 4.8 pg/mL during treatment with 160 and 300 µg of testosterone per day, respectively (normal range,1.3 to 6.8 pg/mL. Despite an appreciable placebo response, the higher testosterone dose resulted in further increases in scores for frequency of sexual activity and pleasure-orgasm in the Brief Index of Sexual Functioning for Women (P = 0.03 for both comparisons with placebo). At the higher dose, the percentages of women who had sexual fantasies, masturbated, or engaged in sexual intercourse at least once a week increased two to three times from base line. The positive-well-being, depressed-mood, and composite scores of the Psychological Well-Being Index also improved at the higher dose (P = 0.04, P = 0.04, respectively, for the comparison with placebo), but the scores on the telephone-based diary did not increase significantly.

In another embodiment of the present invention, testosterone therapy is used in conjunction with estrogen therapy. Studies have shown that testosterone and estrogen replacement resulted in increased sexual desire, frequency of sexual fantasies, sexual arousal, and coital or orgasmic frequency compared to those given estrogen alone or a placebo reported that women receiving estrogen plus testosterone experienced more increased libido, activity, satisfaction, pleasure, fantasy, orgasm, and relevancy as compared to women receiving estrogen alone. Treatment with premarin and methyltestosterone resulted in significantly increased reports of pleasure from masturbation. Treatment with estrogen and methyltestosterone similarly results in increased sexual interest. Most recently, it has been found that transdermal testosterone treatment in women after oophorectomy improved sexual function and psychological well-being. It is contemplated that testosterone administration alone will have therapeutic benefits if given without estrogen. For example, women with hypothalamic amenorrhea show increased vaginal vasocongestion with testosterone treatment compared to a placebo.

In still another embodiment of the present invention, the methods, kits, combinations, and composition are useful in treating decreased bone density in a woman. Another physiologic parameter linked to testosterone administration in women is decreased bone mineral density. Several correlational studies have shown that increased testosterone concentrations are associated with increased bone mineral density. It has been found that higher bioavailable testosterone levels were associated with higher bone mineral density in the ultradistal radius in women. Women having polycystic ovary syndrome had neck bone mineral density positively correlated to free testosterone levels. Upper body bone mineral density had significant correlation with testosterone. A cross-sectional analysis of sex hormone concentrations and bone mineral density in women recruited for a prospective study of risk factors for osteoporosis and found a significant positive correlation between testosterone and bone mineral density. Another study involved an age-stratified sample of 304 women and found a correlation coefficient between bone mineral density and testosterone as shown below in Table 5:

**Table 5: Correlational Coefficients between Testosterone and Bone Mineral Density***

| | **Total** **Testosterone** | **Bioavailable** **Testosterone** |
|---|---|---|
| **Total body** | 0.22 | 0.22 |
| **Lateral spine** | 0.27 | 0.29 |
| **Proximal femur** | 0.25 | 0.30 |
| **Radius** | 0.27 | 0.28 |

| | | |
|---|---|---|
| *Khosla S. et al., J Clin Endocrinol Metab. 1998 Jul;83(7):2266-74. | | |

As with libido and sexual performance, testosterone is often given in conjunction with estrogen in order to prevent bone loss or increase bone mineral density. For example, in a cross sectional study, it was found that subcutaneous estradiol (75 mg) and testosterone (100 mg) prevented osteoporosis and maintained normal bone mineral density in post-menopausal women. In another study the effects of estrogen given alone to those of estrogen plus androgen therapy in post-menopausal women. While the estrogen-only group had a reduction in serum markers of bone formation, women treated with combined estrogen and testosterone had increased bone formation markers. Similarly, it has been shown that estrogen and testosterone replacement with implant pellets increases bone mass more than estrogen implants alone, increased bone mineral density by 5.7% in the spine and 5.2% in the neck femur region. Treatment with estrogen and methyltestosterone similarly results in increased spine and hip bone mineral density. Also, it has been reported that orally given estrogens and methyltestosterone prevented bone loss and increased bone mineral density in the spine and hip.

In another embodiment of the present invention, the methods, kits, combinations, and composition are useful in treating body composition of a woman. Testosterone has been linked to improved body composition in women. Testosterone is positively correlated to body mass index and exogenous androgens influenced body composition and regional body fat distribution in obese post-menopausal women. Other researchers have found an increase in fat-free mass and a reduced fat mass to fat free mass ratio in postmenopausal women treated with concurrent estrogen-testosterone therapy. Thus, administration of testosterone to normal women or those having testosterone deficiencies may have a therapeutic improvement in body composition.

In still another embodiment of the present invention, the methods, kits, combinations, and composition are useful in treating or preventing human immunodeficiency virus wasting syndrome in a woman. In recent years, researchers have found that testosterone administration to women infected with human immunodeficiency virus may treat or prevent human immunodeficiency virus wasting syndrome. It has been found that lower free testosterone levels in human immunodeficiency virus-infected women using a tracer analog method. For example, testosterone replacement in a patch delivering 150 ug/day of testosterone to human immunodeficiency virus-infected women had a 4% increase in body weight over 12 weeks. In addition, the patients had an improved quality of life. Thus, testosterone administration can be used as a method of preventing wasting in women suffering from acquired immunodeficiency syndrome or related disorders.

In yet another embodiment of the present invention, the methods, kits, combinations, and composition are useful in treating or preventing short-term and long-term memory and other higher-order cognitive functions in a woman. Sex steroids are important for short-term and long-term memory and other higher-order cognitive functions. Postmenopausal women receiving estrogen plus testosterone following oophorectomy had higher scores on two tests of short-term memory, a test of long-term memory, and a test of logical reasoning. It has been reported that the administration of testosterone is associated with better visio-spacial function and verbal skills. Women with high testosterone levels scored higher on special/mathematical tasks than women with low testosterone concentrations. Women with higher Mini-Mental State Examination scores had significantly higher mean total and bioavailable testosterone concentrations. Testosterone levels are also related to verbal fluency. Again, the benefits of testosterone administration on cognitive parameters may be optimized by concurrent estrogen administration. For example, subcutaneous implants of oestradiol (40 mg) and testosterone (100 mg) have shown increases in concentration.

In one embodiment of the present invention, the methods, kits, combinations, and compositions are useful in treating or preventing a mood or self-esteem disorder in a woman. Parameters associated with testosterone serum levels in women are mood and self-esteem. Menopausal women who received both estrogen and testosterone felt more composed, elated, and energetic than those who were given estrogen alone. Similarly, testosterone concentrations are positively correlated to self-esteem. Thus, it is contemplated that testosterone therapy will improve mood when used alone or in conjunction with estrogen.

In another embodiment of the present invention, the methods, kits, combinations, and composition are useful in increasing muscle size and performance in a woman. Androgens and anabolic steroids have long since been used to increase muscle size and performance in men. Researchers have recently also found that testosterone is an important determinant of greater muscle size in women with polycystic ovary syndrome. Thus, administration of testosterone to a normal or testosterone deficient woman may be useful for improving muscle mass and performance.

Many of the symptoms described above fall under the umbrella of what is commonly considered to be premenstrual syndrome (PMS). In general, lower levels of testosterone throughout the menstrual cycle have been reported in women who suffer from premenstrual syndrome compared with controls. Testosterone replacement is currently used as a management of premenstrual syndrome in the United Kingdom and Austalia. Managing premenstrual syndrome with oestradiol/testosterone implants resulted in improvements in libido, enjoyment of sex, and tiredness. Thus, it is contemplated that the methods, kits, combinations, and compositions of the present invention can be useful in treating premenstrual syndrome in a woman, especially in conjunction with estrogen administration.

In one embodiment of the present invention, the methods, kits, combinations, and composition are useful in suppressing both cell-mediated and humoral immune responses in a woman. Androgens appear to suppress both cell-mediated and humoral immune responses. Many researchers have advocated increasing testosterone levels in women as protective against autoimmune disease, such as rheumatoid arthritis. Testosterone administration therefore is contemplated to be effective in treating a woman with such disorders.

Toxicity and therapeutic efficacy of the therapeutic agents of the present invention can be determined by standard pharmaceutical procedures, for *example,* for determining LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic induces are preferred. While compounds that exhibit toxic side effects may be used, care should be taken to design a delivery system that targets such compounds to the site of affected tissue in order to minimize potential damage to uninfected cells and, thereby, reduce side effects.

The active agents of the present invention may be administered, if desired, in the form of salts, esters, amides, enantiomers, isomers, tautomers, prodrugs, derivatives and the like, provided the salt, ester, amide, enantiomer, isomer, tautomer, prodrug, or derivative is suitable pharmacologically, that is, effective in the present methods, kits, combinations, and compositions. Salts, esters, amides, enantiomers, isomers, tautomers, prodrugs and other derivatives of the active agents may be prepared using standard procedures known to those skilled in the art of synthetic organic chemistry and described, for example, by J. March, Advanced Organic Chemistry; Reactions, Mechanisms and Structure, 4th Ed. (New York: Wiley-Interscience, 1992). For example, acid addition salts are prepared from the free base using conventional methodology, and involves reaction with a suitable acid. Generally, the base form of the drug is dissolved in a polar organic solvent such as methanol or ethanol and the acid is added thereto. The resulting salt either precipitates or may be brought out of solution by addition of a less polar solvent. Suitable acids for preparing acid addition salts include both organic acids, for example, acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, for example, hydrochloric acid, hydrobromic acid, sulfuric acid, nitric acid, phosphoric acid, and the like. An acid addition salt may be reconverted to the free base by treatment with a suitable base. Particularly preferred acid addition salts of the active agents herein are halide salts, such as may be prepared using hydrochloric or hydrobromic acids. Particularly preferred basic salts here are alkali metal salts, for example, the sodium salt, and copper salts. Preparation of esters involves functionalization of hydroxyl and/or carboxyl groups which may be present within the molecular structure of the drug. The esters are typically acylsubstituted derivatives of free alcohol groups, that is, moieties that are derived from carboxylic acids of the formula RCOOH where R is alkyl, and preferably is lower alkyl. Esters can be reconverted to the free acids, if desired, by using conventional hydrogenolysis or hydrolysis procedures. Amides and prodrugs may also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine. Prodrugs are typically prepared by covalent attachment of a moiety, which results in a compound that is therapeutically inactive until modified by an individual's metabolic system.

The therapeutic agents of the present invention can be formulated as a single pharmaceutical composition containing at least one therapeutic agent, or as independent multiple pharmaceutical compositions where each composition contains at least one therapeutic agent. Pharmaceutical compositions according to the present invention include those compositions with at least one therapeutic agent formulated for percutaneous administration. Percutaneous administration includes transdermal delivery systems that include patches, gels, tapes and creams, and can contain excipients such as alcohols, penetration enhancers, and thickeners, as well as solubilizers (for example propylene glycol, bile salts, and amino acids), hydrophilic polymers (for example, polycarbophil and polyvinylpyrolidone), and adhesives and tackifiers (for example, polyisobutylenes, silicone-based adhesives, acrylates and polybutene).

The therapeutic agents of the present invention can then be administered percutaneously in dosage unit formulations containing conventional nontoxic pharmaceutically acceptable carriers, adjuvants, and vehicles as desired. The compounds of the present invention can be administered by any conventional means available for use in conjunction with pharmaceuticals, either as individual therapeutic compounds or as a combination of therapeutic compounds.

The compositions of the present invention can be administered for treating, preventing, or reducing the risk of developing a testosterone deficiency in a mammal by any means that produce contact of these compounds with their site of action in the body, for example in the ileum, the plasma, or the liver of a mammal.

Additionally, the methods, kits, combinations, and compositions of the present invention may optionally include salts, emollients, stabilizers, antimicrobials, fragrances, and propellants.

In another embodiment of the present invention, the therapeutic agents come in the form of kits or packages containing testosterone. Illustratively, the kits or packages contain testosterone in a dosage form suitable for percutaneous administration, for example, a gel or a patch, in amounts for the proper dosing of the drugs. The therapeutic agents of the present invention can be packaged in the form of kits or packages in which the daily (or other periodic) dosages are arranged for proper sequential or simultaneous administration. The present invention further provides a kit or package containing a plurality of dosage units, adapted for successive daily administration, each dosage unit comprising at least one of the therapeutic agents of the present invention. This drug delivery system can be used to facilitate administering any of the various embodiments of the therapeutic compositions. In one embodiment, the system contains a plurality of dosages to be to be administered daily or weekly via percutaneous administration. The kits or packages also contain a set of instructions for the patient.

The present methods, kits, combinations, and compositions can also be used in "combination therapy" with another steroid or pharmaceutical agent that increases testosterone levels in a mammal, or, as mentioned above, with an estrogenic hormone.

A class of steroids or pharmaceutical agents that increases testosterone levels in a mammal useful in the methods, kits, combinations, and compositions of the present invention include compounds that inhibit the synthesis of the sex hormone binding globulin. Sex hormone binding globulin is a serum protein, and is known to bind to testosterone and estradiol, effecting the biological activity of these hormones. Specific compounds of interest that inhibit the synthesis the sex hormone binding globulin include but are not limited to methyltestosterone and fluoxymesterone, and all salts, esters, amides, enantiomers, isomers, tautomers, prodrugs and derivatives of these compounds. Methyltestosterone is currently available in various formulations including those available orally, for example ANDROID® and TESTRED®. Fluoxymesterone is also currently available in various formulations including those available orally, for example HALOSTESTIN®. Combinations of the above mentioned compounds can be used.

While not wishing to be bound by theory, it is believed that methyltestosterone decreases hepatic synthesis of endogenous proteins like sex hormone binding globulin. This decrease in synthesis produces a decline in blood concentrations of sex hormone binding globulin, which is the primary means of endogenous hormone transport. The decrease in sex hormone binding globulin subsequently causes an increase in free-hormone concentration for binding at the receptor. Transdermal application of an androgen, for example, testosterone, or an estrogen, for example, estradiol, bypasses first-pass metabolism and can provide a means of increasing hormone concentrations in the bloodstream. Thus, when used in combination, methyltestosterone and percutaneously administered testosterone (and optionally estradiol) produce a greater therapeutic effect and provide a means of increasing hormone concentrations in the bloodstream. Methyltestosterone and testosterone (and optionally estradiol) produce a greater therapeutic effect than either entity alone because the decrease in hormone binding ability is coupled with an increased hormone bioavailability, producing higher free-hormone concentrations that would be produced by testosterone alone.

In another embodiment of the present invention, the estrogenic hormone that can be used in conjunction with the methods, kits, combinations, and composition is the naturally occurring estrogen 17 beta-estradiol (beta-estradiol; 1, 3, 5(10)-estratriene-3, 17 beta-diol). Other estrogenic steroid hormones can be used in partial or complete replacement of 17 beta-estradiol, for example, an ester which is biologically compatible and can be absorbed effectively transdermally. The estradiol esters can be, illustratively estradiol-3,17-diacetate; estradiol-3-acetate; estradiol-17-acetate; estradiol-3,17-divalerate; estradiol-3-valerate; estradiol-17-valerate; 3-mono, 17-mono and 3,17-dipropionate esters, corresponding cypionate, heptanoate, benzoate and the like esters; ethynil estradiol; estrone and other estrogenic steroids and salts, enantiomers, isomers, tautomers, prodrugs and derivatives thereof that are possible to administer by transdermal route. Other estrogen-related compounds that may be used in the methods, kits, combinations, and compositions of the present invention include, but are not limited to conjugated estrogens (including estrone sulfate, equilin, and 17-.alpha.-dihydroequilin), estradiol valerate, estriol, estrone, estrone sulfate, estropipate, ethinyl estradiol, mestranol, and all salts, esters, amides, enantiomers, isomers, tautomers, prodrugs and derivatives of these compounds.

Estrogenic hormones are currently available in various formulations including, but not limited to those available as a cream, pessary, vaginal ring, vaginal tablet, transdermal preparation, gel, and oral tablet. Examples of vaginal creams include PREMARIN® (conjugated estrogen), ORTHO DIENOSTEROL® (dienosterol), and OVESTIN® (estriol). Available pessary formulations include ORTHO-GYNEST® (estriol), and TAMPOVAGAN® (stilbestrol). An example of a vaginal ring formulation is ESTRING® (estradiol), and an example of a vaginal tablet is VAGIFEM® (estradiol). Available transdermal estrogen preparations containing estradiol include ERC ALORA®, CLIMARA®, DERMESTRIL®, ESTRADERM®, ESTRADERM® TTS, ESTRADERM® MX, EVOREL®, FEMATRIX®, FEMPATCH®, FEMSEVEN®, MENOREST®, PROGYNOVA® TS, and VIVELLE®. Estrogen gels containing estradiol include ESTRAGEL (under development by Applicant), and SANDRENA®. Estradiol is also available formulated as an implant pellet, for example, ESTRADIOL IMPLANT®. Tablet formulations include PREMARIN® (conjugated estrogen), ESTRATAB® (esterified estrogen), ESTRATEST® (esterified estrogen, methyltestosterone), MENEST® (esterified estrogen), CLIMAGEST®, (estradiol), CLIMAVAL® (estradiol), ELLESTE SOLO® (estradiol), ESTRACE® (estradiol), PROGYNOVA® (estradiol), ZUMENON® (estradiol), HORMONIN® (estradiol, estrone, estriol), HARMOEN® (estrone), OGEN® (estropipate), and ORTHO-EST® (estropipate).

Combinations of the above mentioned estrogenic hormones can be used.

In one embodiment, the estrogenic hormone is formulated for percutaneous administration in a hydroalcoholic gel. The gel comprises one or more lower alcohols, a penetration enhancing agent, a thickener, and water. Additionally, the estrogenic gel optionally includes salts, emollients, stabilizers, antimicrobials, fragrances, and propellants.

Illustratively, the estrogenic gel is comprised of the following substances as shown below in Table 6, in approximate amounts.

| **Table 6: Composition of ESTRAGEL** | |
|---|---|
| **SUBSTANCE** | **AMOUNT (w/w)** **PER 100g OF GEL** |
| 17-beta-oestradiol | 0.06 g |
| Carbopol 980 | 1.0 g |
| Triethanolamine | 1.35 g |
| Ethanol (95% w/w) | (59 ml) |
| Purified water (qsf) | 100 g |

One skilled in the art will appreciate that the constituents of this formulation may be varied in amounts yet continue to be within the spirit and scope of the present invention. For example, the composition may contain about 0.1 to about 10.0 g of estradiol, about 0.1 to about 5.0 g CARBOPOL, about 0.1 to about 5.0 g triethanolamine, and about 30.0 to about 98.0 g ethanol.

The phrase "combination therapy" embraces the administration of a steroid in the testosterone synthesis pathway in conjunction with another steroid or pharmaceutical agent that increases testosterone levels in a mammal, or with an estrogenic hormone, as part of a specific treatment regimen intended to provide a beneficial effect from the co-action of these therapeutic agents for the treatment of a testosterone deficient disorder in a mammal. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days, weeks, months or years depending upon the combination selected). "Combination therapy" generally is not intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, where each therapeutic agent is administered at a different time, as well as administration of these therapeutic agents, or at least two of the therapeutic agents, in a substantially simultaneous manner. Substantially simultaneous administration can be accomplished, for example, by administering to the subject a single gel having a fixed ratio of each therapeutic agent or in multiple, single capsules, tablets, or gels for each of the therapeutic agents. Sequential or substantially simultaneous administration of each therapeutic agent can be effected by any appropriate route including, but not limited to, oral routes, percutaneous routes, intravenous routes, intramuscular routes, and direct absorption through mucous membrane tissues. The therapeutic agents can be administered by the same route or by different routes. For example, a first therapeutic agent of the combination selected may be administered orally, while the other therapeutic agents of the combination may be administered percutaneously. Alternatively, for example, all therapeutic agents may be administered percutaneously, or all therapeutic agents may be administered intravenously, or all therapeutic agents may be administered intramuscularly, or all therapeutic agents can be administered by direct absorption through mucous membrane tissues. The sequence in which the therapeutic agents are administered is not narrowly critical. "Combination therapy" also can embrace the administration of the therapeutic agents as described above in further combination with other biologically active ingredients, such as, but not limited to, agents for improving sexual performance or increasing, and non-drug therapies, such as, but not limited to, surgery.

The therapeutic compounds which make up the combination therapy may be a combined dosage form or in separate dosage forms intended for substantially simultaneous oral administration. The therapeutic compounds that make up the combination therapy may also be administered sequentially, with either therapeutic compound being administered by a regimen calling for two step administration. Thus, a regimen may call for sequential administration of the therapeutic compounds with spaced-apart administration of the separate, active agents. The time period between the multiple administration steps may range from, for example, a few minutes to several hours to days, depending upon the properties of each therapeutic compound such as potency, solubility, bioavailability, plasma half-life and kinetic profile of the therapeutic compound, as well as depending upon the effect of food ingestion and the age and condition of the patient. Circadian variation of the target molecule concentration may also determine the optimal dose interval. The therapeutic compounds of the combined therapy whether administered simultaneously, substantially simultaneously, or sequentially, may involve a regimen calling for administration of one therapeutic compound by oral route and another therapeutic compound by percutaneous route. Whether the therapeutic compounds of the combined therapy are administered orally, by inhalation spray, rectally, topically, buccally (e.g., sublingual), or parenterally (e.g., subcutaneous, intramuscular, intravenous and intradermal injections, or infusion techniques), separately or together, each such therapeutic compound will be contained in a suitable pharmaceutical formulation of pharmaceutically-acceptable excipients, diluents or other formulations components. Examples of suitable pharmaceutically-acceptable formulations containing the therapeutic compounds are given above. Additionally, drug formulations are discussed in, for example, Hoover, John E., Remington's Pharmaceutical Sciences, Mack Publishing Co., Easton, Pennsylvania 1975. Another discussion of drug formulations can be found in Liberman, H.A. and Lachman, L., Eds., Pharmaceutical Dosage Forms, Marcel Decker, New York, N.Y., 1980.

The present invention is further illustrated by the following examples, which should not be construed as limiting in any way. In the below example, it is assumed that normal cycling women produce approximately 300 µg of testosterone per day, and their serum testosterone levels generally range from about 20 ng/dL to about 80 ng/dL averaging about 40 ng/dL. Bilateral oophorectomy in pre-menopausal women reduces testosterone production by approximately 50%, resulting in an average total serum level of approximately 20 ng/dL. From a physiological perspective, testosterone therapy in surgically menopausal women who, for example, experience female sexual dysfunction, is to replace the missing ovarian testosterone production of approximately 150 µg per day and restore the levels of testosterone and its active androgenic metabolite dihydrotestosterone (DHT) to their previous levels within the normal physiological range.

The following examples are provided for exemplification of the present invention and are not intended to be limiting in any way.

### EXAMPLES

### Example 1. Dosage of Testosterone in a Female after Bilateral Oophorectomy

In one embodiment of the present invention, the methods, kits, combinations, and compositions are comprised of a percutaneously deliverable testosterone formulation. In this example, testosterone is formulated as a gel for transdermal administration as described above in Table 3 (RELIBRA).

In a prophetic example, 24 pre-menopausal women who have undergone bilateral oophorectomy are randomized to receive: (a) 0.17 g/day of RELIBRA, which delivers 1.7 mg/day of testosterone to the skin of which about 0.1 mg, is absorbed, for 30 days; or (b) 0.25 g/day of RELIBRA, which delivers 2.5 mg/day of testosterone to the skin of which about 0.15 mg is absorbed, for 30 days; or (c) 0.5 g/day of RELIBRA, which delivers 5.0 mg/day of testosterone to the skin of which about 0.3 mg is absorbed, for 30 days; or (d) a gel containing a placebo for 30 days. The gel is rubbed onto the clean dry skin of the upper outer thigh and hip once daily. Following application, the gel is allowed to air dry. The patient washes her hands

Applicants expect that from a physiological perspective, all test parameters will show an improvement in female sexual dysfunction over the placebo. Accordingly, Applicant expects that RELIBRA can be applied to improve female sexual dysfunction as compared to placebo in pre-menopausal women who have undergone a bilateral oophorectomy.

### Example 2. Dosage of Testosterone and Methyltestosterone in a Female after Bilateral Oophorectomy

In one embodiment of the present invention, the methods, kits, combinations, and compositions are comprised of a percutaneously deliverable testosterone formulation, and an orally deliverable methyltestosterone formulation. In this example, testosterone is formulated as a gel for transdermal administration as described above in Table 3 (RELIBRA), and methyltestosterone is formulated as a capsule for oral administration and each dosage unit contains 10 mg of methyltestosterone.

In a prophetic example, 24 pre-menopausal women who have undergone bilateral oophorectomy are randomized to receive a daily oral dose of 10 mg or 50 mg methyltestosterone for 30 days, plus: (a) 0.17 g/day of RELIBRA, which delivers 1.7 mg/day of testosterone to the skin of which about 0.1 mg, is absorbed, for 30 days; or (b) 0.25 g/day of RELIBRA, which delivers 2.5 mg/day of testosterone to the skin of which about 0.15 mg is absorbed, for 30 days; or (c) 0.5 g/day of RELIBRA, which delivers 5.0 mg/day of testosterone to the skin of which about 0.3 mg is absorbed, for 30 days; or (d) a gel containing a placebo for 30 days. The gel is rubbed onto the clean dry skin of the upper outer thigh and hip once daily. Following application, the gel is allowed to air dry. The patient washes her hands.

Applicants expect that from a physiological perspective, all test parameters will show an improvement in female sexual dysfunction over the placebo. Accordingly, Applicant expects that RELIBRA can be administered in conjunction with methyltestosterone to improve female sexual dysfunction as compared to placebo in pre-menopausal women who have undergone a bilateral oophorectomy.

### Example 3. Dosage of Testosterone and Estrogen in a Female after Bilateral Oophorectomy

In one embodiment of the present invention, the methods, kits, combinations, and compositions are comprised of a percutaneously deliverable testosterone formulation, and a non-orally deliverable estrogen. In this example, testosterone is formulated as a gel for transdermal administration as described above in Table 3 (RELIBRA), and estradiol is formulated as a gel for transdermal administration as described above in Table 5 (ESTRAGEL).

In a prophetic example, 24 pre-menopausal women who have undergone bilateral oophorectomy are randomized to receive a daily dose of 5 g or 10 g ESTRAGEL for 30 days, plus: (a) 0.17 g/day of RELIBRA, which delivers 1.7 mg/day of testosterone to the skin of which about 0.1 mg, is absorbed, for 30 days; or (b) 0.25 g/day of RELIBRA, which delivers 2.5 mg/day of testosterone to the skin of which about 0.15 mg is absorbed, for 30 days; or (c) 0.5 g/day of RELIBRA, which delivers 5.0 mg/day of testosterone to the skin of which about 0.3 mg is absorbed, for 30 days; or (d) a gel containing a placebo for 30 days. The gel is rubbed onto the clean dry skin of the upper outer thigh and hip once daily. Following application, the gel is allowed to air dry. The patient washes her hands.

Applicants expect that from a physiological perspective, all test parameters will show an improvement in female sexual dysfunction over the placebo. Accordingly, Applicant expects that RELIBRA can be administered in conjunction with estradiol to improve female sexual dysfunction as compared to placebo in pre-menopausal women who have undergone a bilateral oophorectomy.

### Example 4. Combination Testosterone and Estrogen Gel

| **Substance** | **Amount (w/w) per 100g of Gel** |
|---|---|
| Testosterone | 1.0g (or about 0.5g) |
| 17-beta-oestradiol | 0.06g (or about 0.10g) |
| Carbopol 980 | 1.0g |
| Triethanolamine | 1.35g |
| Isopropyl myristate | 0.50g |
| 0.1 N NaOH | 4.72g |
| Ethanol (95% w/w) | 72.5g |
| Purified Water (qsf) | 100g |

The gel is rubbed onto the clean dry skin of the upper outer thigh and hip once daily. Following application, the gel is allowed to air dry. The patient washes her hands. Application of the gel results in an increased testosterone level having a desirable pharmacokinetic profile similar to that in normal women. The gel is thus useful for treating a number of conditions or diseases in women.

The contents of all cited references throughout this application are hereby expressly incorporated by reference. The practice of the present invention will employ, unless otherwise indicated, conventional techniques of pharmacology and pharmaceutics, which are within the skill of the art.

Although the invention has been described with respect to specific embodiments and examples, it should be appreciated that other embodiments utilizing the concept of the present invention are possible without departing from the scope of the invention. The present invention is defined by the claimed elements, and any and all modifications, variations, or equivalents that fall within the true spirit and scope of the underlying principles.

The invention also highlights the following items:
1. A method of treating, preventing or reducing the risk of developing a testosterone deficient disorder in a female mammal in need thereof, comprising administering to the female mammal a testosterone deficient disorder-effective amount of a percutaneously deliverable pharmaceutically-acceptable steroid in the testosterone synthetic pathway in a hydroalcoholic gel.
2. The method of item 1 wherein the steroid is at least one of testosterone, androstenedione, androstenediol, dehydroepiandrosterone, prenenolone, or dihydrotestosterone.
3. The method of item 2 wherein the steroid is testosterone.
4. The method of item 1 wherein the hydroalcoholic gel further comprises at least one of a lower alcohol, a penetration enhancer, or a thickener.
5. The method of item 4 wherein the lower alcohol is at least one of ethanol, or 2-propanol, or mixtures thereof.
6. The method of item 5 wherein the enhancer is isopropyl myristate.
7. The method of item 6 wherein the thickener is CARBOPOL®.

## Claims

1. Percutaneously deliverable pharmaceutically-acceptable steroid in the testosterone synthetic pathway in a hydroalcoholic gel for use in the treatment, the prevention or the reduction of the risk of developing a testosterone deficient disorder in a female mammal.

2. The percutaneously deliverable pharmaceutically-acceptable steroid in the testosterone synthetic pathway in a hydroalcoholic gel according to claim 1, wherein the steroid is selected from the group consisting of testosterone, androstenedione, androstenediol, dehydroepiandrosterone, prenenolone, and dihydrotestosterone.

3. The percutaneously deliverable pharmaceutically-acceptable steroid in the testosterone synthetic pathway in a hydroalcoholic gel according to claim 2, wherein the steroid is testosterone.

4. The percutaneously deliverable pharmaceutically-acceptable steroid in the testosterone synthetic pathway in a hydroalcoholic gel according to any of the preceding claims, wherein the hydroalcoholic gel comprises a lower alcohol, a penetration enhancer, and a thickener.

5. The percutaneously deliverable pharmaceutically-acceptable steroid in the testosterone synthetic pathway in a hydroalcoholic gel according to claim 4, wherein the lower alcohol is at least one of ethanol, or 2-propanol, or mixtures thereof.

6. The percutaneously deliverable pharmaceutically-acceptable steroid in the testosterone synthetic pathway in a hydroalcoholic gel according to one of claim 4 or claim 5, wherein the enhancer is isopropyl myristate or isostearic acid.

7. The percutaneously deliverable pharmaceutically-acceptable steroid in the testosterone synthetic pathway in a hydroalcoholic gel according to any of claims 4 to 6, wherein the thickener is CARBOPOL®.

8. The percutaneously deliverable pharmaceutically-acceptable steroid in the testosterone synthetic pathway in a hydroalcoholic gel according to any of claims 1 to 7, wherein said percutaneously deliverable pharmaceutically-acceptable steroid in the testosterone synthetic pathway in a hydroalcoholic gel is used in conjunction with another steroid or pharmaceutical agent that increases testosterone levels in a mammal.

9. The percutaneously deliverable pharmaceutically-acceptable steroid in the testosterone synthetic pathway in a hydroalcoholic gel according to claim 8, wherein said steroid or pharmaceutical agent that increases testosterone levels in a mammal is a compound that inhibits the synthesis of the sex hormone binding globulin, preferably methyltestosterone or fluoxymesterone.

10. The percutaneously deliverable pharmaceutically-acceptable steroid in the testosterone synthetic pathway in a hydroalcoholic gel according to any of claims 1 to 7, wherein said percutaneously deliverable pharmaceutically-acceptable steroid in the testosterone synthetic pathway in a hydroalcoholic gel is used in conjunction with an estrogenic hormone.

11. The percutaneously deliverable pharmaceutically-acceptable steroid in the testosterone synthetic pathway in a hydroalcoholic gel according to claim 10, wherein said percutaneously deliverable pharmaceutically-acceptable steroid in the testosterone synthetic pathway in a hydroalcoholic gel is used in conjunction with estradiol.

12. The percutaneously deliverable pharmaceutically-acceptable steroid in the testosterone synthetic pathway in a hydroalcoholic gel according to any of claims 1 to **11,** wherein it is to be administered to a woman selected from the group consisting of a oophorectomized/hysterectomized woman, a post-menopausal woman on estrogen replacement therapy, a woman on oral contraceptives, a woman with an ovariectomy, a woman with premature ovarian failure, a woman with adrenal dysfunction, a woman with corticosteroid-induced adrenal suppression, a woman with panhypopituitarism, a woman with primary adrenal insufficiency, and a woman experiencing chronic illness, such as systemic lupus erythematosis, rheumatoid arthritis, human immunodeficiency virus (HIV) infection, chronic obstructive lung disease, and end stage renal disease.

13. A method for producing high free sex hormone concentrations in a female mammal in need thereof, comprising administering to the female mammal a percutaneously deliverable pharmaceutically-acceptable steroid in the testosterone synthetic pathway in a hydroalcoholic gel in conjunction with methyltestosterone.

14. A method for increasing sexual desire, frequency of sexual fantasies, sexual arousal and coital or orgasmic frequency in a female mammal, comprising administering to the female mammal a percutaneously deliverable pharmaceutically-acceptable steroid in the testosterone synthetic pathway in a hydroalcoholic gel in conjunction with an estrogenic hormone.

15. A method for treating premenstrual syndrome in a female mammal in need thereof, comprising administering to the female mammal a percutaneously deliverable pharmaceutically-acceptable steroid in the testosterone synthetic pathway in a hydroalcoholic gel in conjunction with an estrogenic hormone.
